# EUROPEAN PATENT SPECIFICATION

(11) **EP 2 023 950 B1**
(45) Date of publication and mention of the grant of the patent: **11.07.2012**
(21) Application number: 07776833.1
(22) Date of filing: 07.05.2007
(51) Int. Cl.: A61K 38/22

(54) **Urodilatin cancer treatment**
Krebsbehandlung mit Urodilatin
Traitement du cancer par l'urodilatine

(30) Priority: 05.05.2006 US 746562 P
(43) Date of publication of application: 18.02.2009
(73) Proprietor: University of South Florida, Tampa, FL 33612 (US); United States Department of Veterans Affairs, Washington, DC 20420 (US)
(72) Inventor: VESELY, David, L., Tampa, FL 33647 (US)
(74) Representative: Chaillot, Geneviève
(86) International application number: PCT/US2007/011013
(87) International publication number: WO 2007/130672

(56) References cited:
- WO-A2-2004/083236
- US-A1- 2004 229 784
- US-A1- 2005 176 641
- VESELY B A ET AL: "Four peptide hormones' specific decrease (up to 97%) of human prostate carcinoma cells" EUROPEAN JOURNAL OF CLINICAL INVESTIGATION, vol. 35, no. 11, November 2005 (2005-11), pages 700-710, XP002587138 ISSN: 0014-2972
- VESELY DAVID L: "ATRIAL NATRIURETIC PEPTIDES: ANTICANCER AGENTS" JOURNAL OF INVESTIGATIVE MEDICINE, LIPPINCOTT WILLIAMS & WILKINS, US, vol. 53, no. 7, 1 November 2005 (2005-11-01), pages 360-365, XP008073768 ISSN: 1081-5589
- SABA SABIHA R ET AL: "Immunocytochemical localization of atrial natriuretic peptide, vessel dilator, long-acting natriuretic peptide, and kaliuretic peptide in human pancreatic adenocarcinomas." THE JOURNAL OF HISTOCHEMISTRY AND CYTOCHEMISTRY : OFFICIAL JOURNAL OF THE HISTOCHEMISTRY SOCIETY AUG 2005 LNKD- PUBMED:15879575, vol. 53, no. 8, August 2005 (2005-08), pages 989-995, XP002587139 ISSN: 0022-1554
- VESELY B A ET AL: "Urodilatin and four cardiac hormones decrease human renal carcinoma cell numbers." EUROPEAN JOURNAL OF CLINICAL INVESTIGATION NOV 2006 LNKD- PUBMED:17032349, vol. 36, no. 11, 9 October 2006 (2006-10-09), pages 810-819, XP002587140 ISSN: 0014-2972

## Description

### FIELD OF INVENTION

This invention relates to a new treatment of cancer, specifically, Urodilatin, a peptide formed in the kidney, has significant anticancer effects of killing up to 81% of cancer cells within 24 hours.

### BACKGROUND OF THE INVENTION

Approximately 36,160 patients in the United States were diagnosed with renal carcinomas with an estimated 12,600 deaths in 2005 [1]. The most common cancer of the kidney is renal-cell carcinoma which accounts for approximately 85% of renal cancers (2-4). With the high incidence of deaths from renal-cell carcinomas with current therapy and the high rate of recurrence of renal-cell carcinoma after nephrectomy, demands an aggressive search for new therapeutic agents [3-9].

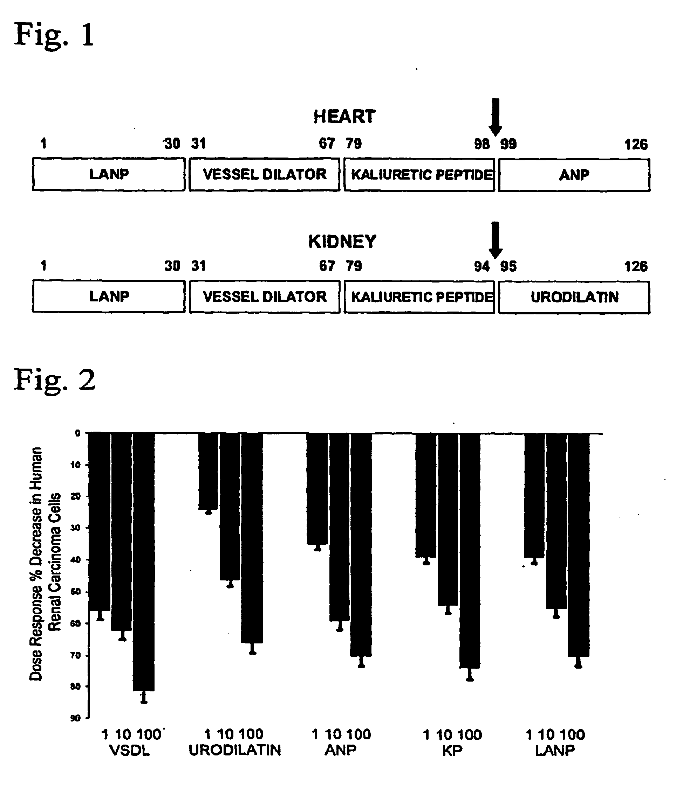

Cardiac natriuretic peptides consist of a family of peptides that have significant anticancer effects on human breast, prostate, colon and pancreatic adenocarcinomas as well as small-cell and squamous lung carcinoma cells [10-16]. Within the 126 amino acid (a.a.) ANP prohormone synthesized in the heart are four peptide hormones i.e., long acting natriuretic peptide (LANP), vessel dilator, kaliuretic peptide and atrial natriuretic peptide (ANP) (Fig. 1), whose main known biological properties in addition to anticancer effects are blood pressure regulation and maintenence of plasma volume in animals [17-23] and humans [24-27].

Urodilatin is a peptide hormone formed by a differential processing of the ANP prohormone in the kidney, as opposed to all other tissues, where instead of cleaving the 126 a.a. prohormone between a.a. 98 and 99 to form ANP and kaliuretic peptide it cleaves this prohormone between a.a. 95 and 96 [28-32] (Fig. 1). The cleavage of the ANP prohormone in the kidney results in 4 **a.a.** from the C-terminal end of kaliuretic peptide. (i.e., threonine-alanine-proline-arginine) being attached to the N-terminus of ANP with the resultant peptide called urodilatin [28-32]. It is important to note in this regard that the amino acids in urodilatin are identical to the four C-terminal a.a. of kaliuretic peptide and identical to all the a.a. in the ANP portion of this kidney peptide [28-32]. Urodilatin has never been investigated for its possible anticancer effects.

The present investigation was designed to determine if urodilatin has anticancer effects. The hypothesis behind this investigation is that urodilatin consists of 4 a.a. of kaliuretic peptide attached to ANP [28-32], both of which have anticancer effects [10-16) and, thus, urodilatin with identical a.a. contributed from these two cardiac peptides might have anticancer effects also (See U.S. Patent No. 6943147 incorporated by reference herein). Since the cardiac natriuretic peptides have antigrowth effects on malignant tumors of the heart [33], i.e., on tumors in the same organ in which the peptides are synthesized, this is the precedent for studying urodilatin synthesized in the kidney on kidney cancer cells. There has never been an investigation of the cardiac natriuretic peptides on any renal cancer so they were added to the design of this study to compare their effects with urodilatin. In previous studies of the cardiac natriuretic peptides' anticancer effects *in vitro* these peptide hormones were given for four days, with this protocol demonstrating that there was a marked decrease in cancer cell number with no proliferation of the cancer cells for three days after the initial treatment [10,12-16]. The present investigation was also designed to answer the question "can the peptide hormones when only given for 24 hours prevent proliferation of remaining cancer cells when followed for three more days?" It was also investigated whether part of the mechanism of this decrease was owing to inhibition of DNA synthesis in the renal cancer cells. The present investigation further examined if the renal cancer cells have natriuretic peptide receptors (NPR)-A and C- to mediate these peptide hormones' effects, as natriuretic peptide receptors have never been demonstrated on renal cancer cells.

### SUMMARY OF THE INVENTION

The present invention includes the use of urodilatin for the manufacture of a medicament for inhibiting the growth of cancer cells. In a preferred embodiment, said use involves urodilatin in conjunction with at least one other peptide hormone derived selected from the group consisting of atrial natriuretic peptide, long acting natriuretic peptide, vessel dilator, and kaliuretic peptide. In a further preferred embodiment said cancer cells are renal carcinoma cancer cells. The invention also includes the use of a combination of peptide hormones derived from the atrial natriuretic peptide prohormone for the manufacture of a medicament for inhibiting the growth of cancer cells, wherein the combination includes urodilatin. In a preferred embodiment said combination of peptide hormones derived from the atrial natriuretic peptide prohormone is selected from the group consisting of atrial natriuretic peptide, long acting natriuretic peptide, vessel dilator, and kaliuretic peptide. In a further preferred embodiment the cancer cells are renal carcinoma cancer cells.

### BRIEF DESCRIPTION OF THE DRAWINGS

For a fuller understanding of the invention, reference should be made to the following detailed description, taken in connection with the accompanying drawings, in which:
Figure 1. Different translational processing of atrial natriuretic peptide (ANP) prohormone in kidney versus heart resulting in the formation of urodilatin, a peptide synthesized only in the kidney. In the kidney, the ANP prohormone is cleaved between amino acids (a.a.) 94 and 95, rather than between a.a. 98 and 99 as in the heart and other tissues. Four amino acids from the C-terminus of kaliuretic peptide are added to ANP with the resulting peptide being named urodilatin. The amino acids in urodilatin are identical to the amino acids in ANP and identical to the last four C-terminal a.a. of kaliuretic peptide.
Figure 2. Dose-response of urodilatin (URO), kaliuretic peptide (KP), atrial natriuretic peptide (ANP), vessel dilator (VSDL), and long acting natriuretic peptide (LANP) anticancer effects on human renal-cell carcinoma cells. As each increasing concentration of these five peptide hormones there was a significantly (P<0.05) increased decrease in renal cancer cells after 24 hours except between the 1 µM and 10 µM concentration of vessel dilator when evaluated by repeated measures of ANOVA. Vessel dilator caused the same decrease as the other peptide hormones at a 10-fold lower concentration, as observed in this figure (n-60 for each group).

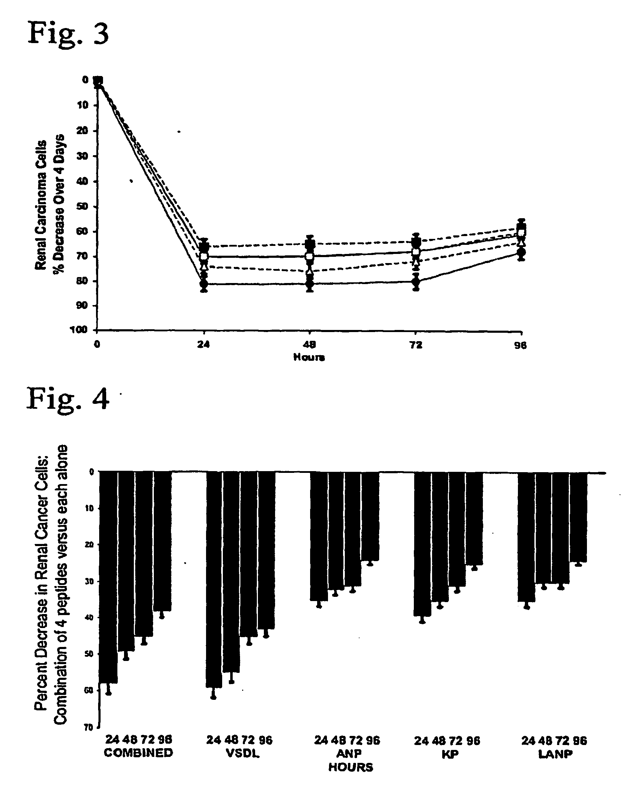

Figure 3. Time course in decrease of human renai-cell carcinoma cell number with 100 µM concentration of urodilatin (■), atrial natriuretic peptide (▲), kaliuretic peptide (Δ), vessel dilator (O), and long acting natriuretic peptide (□) at 24, 48, 72, and 96 hours were significant at P<0.001 compared to placebo-treated (●) renal cancer cells when evaluated by repeated analysis of variance (n=60 for each group).
Figure 4. Comparison of the decrease in renal-cell cancer cell number with the combining of all four peptide hormones from the cardiac ANP gene versus each alone at I µM over a 96 hour period. The combining of the four peptide hormones (i.e., long acting natriuretic peptide (LANP), vessel dilator (VSDL), kaliuretic peptide (KP) and atrial natriuretic peptide (ANP) each at I µM caused a significant (P<0.05) decrease in cancer cell number compared to LANP, ANP and kaliuretic peptide (each at I µM) but this decrease was not significantly greater than that observed with 1 µM of vessel dilator alone when evaluated by repeated analysis of variance (n=60 in each group).

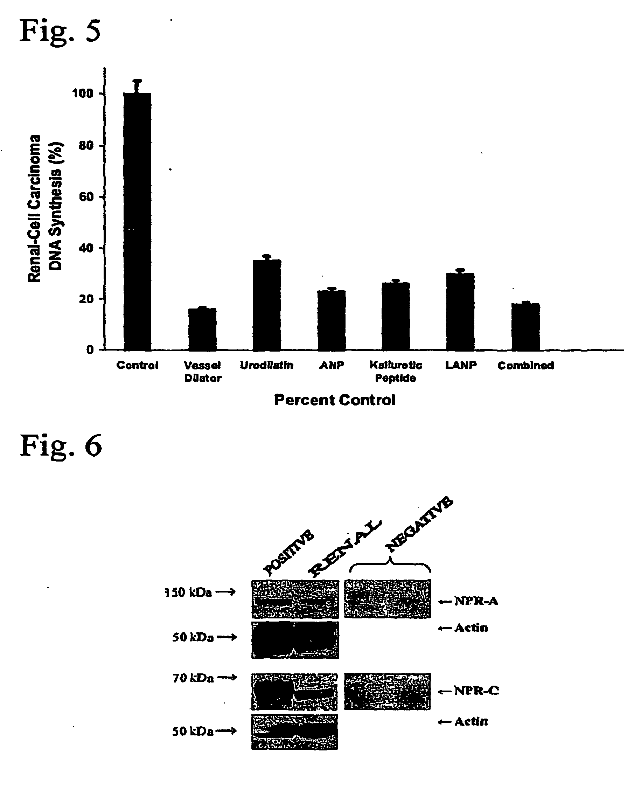

Figure 5. Decrease in DNA synthesis by urodilatin, atrial natriuretic peptide (ANP), kaliuretic peptide, vessel dilator and long acting natriuretic peptide (LANP). The 65 to 84% decrease in DNA synthesis secondary to these five hormones (■) each at 1 µM, was significant (P<0.001) compared to control (i.e., untreated) cells (□) (P<0.001) when evaluated by repeated measures of analysis of variance (ANOVA) (n=30 for each group).
Figure 6. Natriuretic receptors (NPR) A- and C-rcceptors are present in human renal-cell carcinoma cells. Western blot analysis with 1:4000 dilution of R1214 polyclonal antibody directed against the COOH terminus of the natriuretic A-receptor (kindly provided by Dr. David L. Garbers, University of Texas Southwestern, Dallas, Texas) and 1:1000 dilution of Omari antibody to the NPR-C receptor (generously provided by Dr. Kenj Omari, Osaka, Japan). The right-hand graph (upper panel) demonstrates the positive control for the NPR-A receptor and the NPR-A receptor in human renal-cell carcinoma cells (CRL2175). The lower panel right-hand graph demonstrates the NPR-C receptor at 66 kilo Daltons (kDa) in the human renal-cell carcinoma cells as well as the positive control (left panel of figure). The negative controls are in the right panel of these figures. Albumin (bovine serum albumin, BSA) (70 kDa) was used in addition to BIO RAD Precision Plus Protein Dual Color standards to identify the bands corresponding to the NPR-A and NPR-C receptors, respectively. Re-probing with Actin was used as a loading control. The renal carcinoma cells for receptor analysis were scraped from 100 mm dishes in ice cold mammalian protein extraction reagent (M-PER; Pierce; Rockford, IL) containing phosphatase inhibitor (Pierce) and Hae protease inhibitors (Pierce).

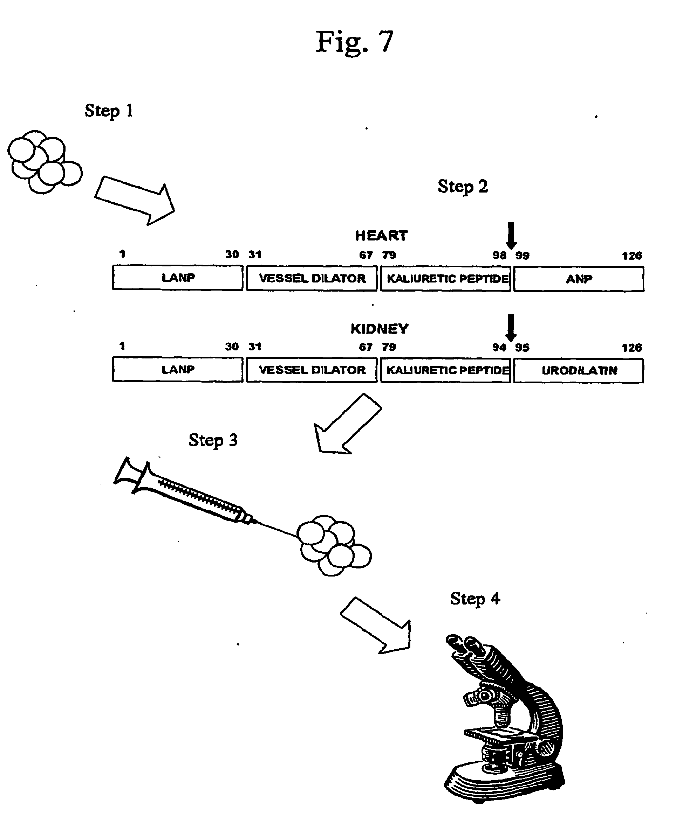

Figure 7. A diagrammatic representation of an embodiment of the invention.

### DETAILED DESCRIPTION

### Renal carcinoma cells

A cell line (ATCC number CRL-2175) of human renal-cell carcinoma cells were purchased from American Type Culture Collection (ATCC, Manassas, VA). This renal cancer cell line was established by A. Leibovitz in 1972 from a renal-cell carcinoma removed from a 75 year old Caucasian man with blood type O Rh+.

### Culture of the renal-cell carcinoma cells

Propagation of these renal cancer cells was in Leibovitz's L-15 medium supplemented with 10% fetal bovine serum (Atlanta Biologicals, Lawrenceville, GA) at a temperature of 37°C in a CO₂ free environment as recommended by the ATCC. Cells were dispensed in new flasks with subculturing two to three times per week.

### Research protocol

After the renal-cell carcinoma cells were subcultured for 24 hours they were then seeded to coverslips in 24-well plates (Nunclon^{™}, Roskilde, Denmark) with I mL of the above media. There were 65,000 cells seeded to each coverslip. After 24 hours, the well plates were washed twice with phosphate-buffered saline to remove the fetal bovine serum. Removal of serum was carried out to completely remove all variables (EGF, etc.) present in serum in order that interpretation of any data obtained would be straightforward. After 24 hours of serum deprivation, media volume was reduced to 250 µL per well with, or without; the respective peptide hormones in dose-response curves with concentrations up to and including 100 µM (1% of this volume). Human renal carcinoma cells were then incubated for various periods of time [24,48,72, and 96 hours]. The number of renal carcinoma cells were then counted with a cell counter (Thomas Scientific^{®}, Swedesboro, NJ) evaluating ten fields of the microscope slide at x40 along the X-axis with an Olympus BH-2 microscope (Atlanta, Georgia). This evaluation was repeated on six separate occasions with the number of renal carcinoma cells reflecting 60 observations for each group, i.e., 60 observations for controls and 60 observations for each of the six groups with respective peptide hormones. The peptide hormones used in this investigation were from Phoenix Pharmaceuticals, Inc., Belmont, CA. In the Results section, the number of cancer cells reported is the number of cells in each individual field. Ten fields were examined on each microscope slide. The results of the ten fields were pooled and the average of the ten fields is illustrated in the Results section.

### Determination of DNA Synthesis

To investigate whether these peptide hormones were inhibiting DNA synthesis, bromodeoxyuridine (BrdU) incorporation [34-38] into the renal carcinoma cells was utilized as previously described from our laboratory [10,12-16]. BrdU was from BD Bioscience, San Jose. California. After 24 hours in culture with 1 µM of LANP, vessel dilator, kaliuretic peptide, ANP, and urodilatin, respectively, or with no peptide hormone (i.e., control), BrdU in a final concentration of 10 µM in the cell culture medium was added for 45 minutes, which is the time in which the cells are in the logarithmic phase of cell proliferation.

### ANP Receptors In Human Renal Carcinoma Cells

Renal cancers have never been examined to determine if they have natriuretic receptors. When it was found that these ANPs decreased the number of human renal-cell cancer cells, It was then evaluated whether renal-cell cancer cells have ANP receptors to mediate these effects. Western blots of the natriuretic peptide receptors (NPR) A- and C- were performed as described previously from our laboratory [10,12-16] using 75 µg of protein extract from human renal-cell cancer cells, measured by using the bicinchoinic acid protein assay kit (Pierce; Rockford, IL), which was loaded onto each lane of a Criterion Precast 7.5% Tris-HCl gel (Bio-Rad; Hercules, CA), separated by electrophoresis (100 volts for 120 min), and then transblotted onto a nitrocellulose membrane (Hybond-C Extra, Amersham Biosciences Corporation, Piscataway, NJ) for 75 min at 100 volts in Towbin buffer.

### RESULTS

### Urodilatin decreases the number of human renal carcinoma cells

The number of renal carcinoma cells after 24 hours without the addition of any of the peptide hormones averaged 89 ± 7 cells per high powered field when ten fields of the coverslip were evaluated at x 40 along the *X*-axis with an Olympus BH₂ microscope. This evaluation was repeated on six separate occasions, with the above number reflecting 60 observations by two independent investigators of the control group and each of the five groups with the addition of one of the peptide hormones. The same number of observations were done in a seventh group where four of the peptides were added together (each at 1 µM) to determine if they may have an additive effect.

Urodilatin in dose-response curves decreased the number of human renal carcinoma cells in 24 hours 24%, 46%, and 66% (down to 30 ± 2 cells) at its 1 µM, 10 µM, and 100 µM concentrations, respectively at 24 hours (Fig. 2).

### Decreased renal-cell carcinoma cell proliferation for two days after the initial 24-hoor exposure to urodilatin

When the renal cell carcinoma cells were followed for longer periods of time i.e., 48, 72, and 96 hours after the 24 hour exposure, urodilatin decreased the number of renal cell carcinoma cells 65%, 64%, and 58% at its 100 µM concentration, 46%, 43%, and 37% at its 10 µM concentration and 17%, 15%, and 10% at I µM concentration (Fig. 3).

### Decreased number of human kidney carcinoma cells by four peptide hormones synthesized by the cardiac ANP gene

The number of human renal carcinoma cells in culture for 24 hours decreased 59%, 62% and 81% (down to 17 ± 4 cancer cells from 89 ± 7 cells) secondary to vessel dilator at its 1 µM, 10 µM, and 100 µM concentrations, respectively (Fig. 2). Thus, at each respective concentration vessel dilator's anticancer effects were stronger than urodilatin's. Dose-response curves revealed that LANP in culture for 24 hours decreased the number of renal carcinoma cells 39%, 55% and 70% (decreased to 26 ± 2 cancer cells) at its 1 µM, 10 µM, and 100 µM concentrations, respectively (Fig. 2). Exposure of the human renal carcinoma cells to kaliuretic peptide resulted in a 39%, 54%, and 74% (23 ± 3 cancer cells) at its 1 µM, 10 µM, and 100 µM concentrations, respectively (Fig. 2). The addition of ANP decreased the number of renal carcinoma cells in 24 hours by 35%, 59% and 70% at its 1 µM, 10 µM, and 100 µM concentrations. Thus, with respect to their ability to inhibit the growth of human renal carcinoma cells when these cells were exposed to identical 1 µM concentrations of these peptide hormones for 24 hours was vessel dilator > LANP > kaliuretic peptide > ANP > urodilatin. When the number of cancer cells was examined immediately after the incubation with the respective peptide hormones, there was no decrease in the number of cancer cells. In the wells with a decreased number of cells secondary to urodilatin and the cardiac hormones there was evidence of cellular debris.

### Decreased renal carcinoma cellular proliferation for two days after initial 24 hour exposure of these peptide hormones

When the renal carcinoma cells were followed for three days after treatment with vessel dilator, LANP, kaliuretic peptide and ANP there was nearly complete inhibition of proliferation of renal carcinoma cells at 48 and 72 hours after the decrease in the number of the renal carcinoma cells at 24 hours by the peptide hormones from the cardiac ANP prohormone gene (Fig. 3). Thus, when exposed to vessel dilator for 24 hours but without exposure to vessel dilator for the next 24 hours, the decrease in number of renal cancer cells at 48 hours was 81%, 66%, and 55% at 100 µM, 10 µM, and I µM of vessel dilator (non-significant difference from the amount of decrease at 24 hours). Likewise at 48 hours the decrease in renal carcinoma cells secondary to kaliuretic peptide was nearly identical to that observed at 24 hours with a 76%, 52%, and 35% decrease at 48 hours with 100 µM, 10 µM, and 1 µM of kaliuretic peptide (non-significant difference from 24 hours). At 48 hours, after an exposure to LANP for only 24 hours, there was a 70%, 50%, 30% decrease in renal cancer cell number at its 100 µM, 10 µM and 1 µM concentrations. Exposure to ANP for 24 hours but without exposure ANP for the next 24 hours resulted in no proliferation of renal carcinoma cells as there was a 70%, 55%, and 32% decrease in renal cancer cells with 100 µM, 10 µM, and 1 µM of ANP (non-significant difference comparing these different concentrations 24 hours and 48 hours). Two days after exposure to the respective peptide hormones (i.e., 72 hours in figure 3 illustrated for 100 µM concentration) there was no proliferation in the remaining renal cancer cells. Thus, with vessel dilator there was an 80%, 64% and 51% decrease while with kaliuretic peptide there was a 71%, 47%, and 31% decrease at 72 hours in renal cancer cells at their 100 µM, 10 µM, and 1 µM concentrations, respectively. LANP caused a 70%, 47%, and 30% decrease while with ANP there was a 68%, 51% and 31% decrease in renal cell cancer numbers at their 100 µM, 10 µM, and 1 µM concentrations at 72 hours.

Three days after exposure to the respective peptide hormones, there was some proliferation of the renal cell carcinoma cells (Fig. 3). Thus, the renal cancer cells that had not been exposed to vessel dilator for three days had a 68%, 55% and 43% decrease in cancer cell number compared to control and with kaliuretic peptide there was a 64%, 40%, and 25% decrease and the decrease secondary to LANP and ANP was 61%, 39%, 24% and 60%, 43% and 24% at their 100 µM, 10 µM, and 1 µM concentrations, respectively. This is an aggressive cancer cell in culture as evidenced by the control number of renal carcinoma cells increasing 77%, 180%, and 280% at 48, 72 and 96 hours compared to 24 hours.

### Combination of LANP, vessel dilator, ANP and kaliuretic peptide does not decrease renal carcinoma cell number more than vessel dilator alone

Combining LANP, vessel dilator, kaliuretic peptide and ANP, each at 1 µM, resulted in a 58% decrease in renal carcinoma cell numbers at 24 hours. Although this 58% decrease was larger than ANP (35%), LANP (35%) and kaliuretic peptide's (39%) decrease in cancer cell number at their I µM concentrations for 24 hours, the combined decrease secondary to these four peptide hormones was not larger than 1 µM of vessel dilator used alone (i.e., 59% decrease) (Fig. 4). At 48 hours the four peptide hormones combined (each at 1 µM) caused a 49% decrease renal cell carcinoma cell number while vessel dilator alone at 1 µM caused a 55% decrease in renal cell cancer number (Fig. 4). At 72 hours there was a 45% decrease when the four peptides were combined while vessel dilator caused a 51% decrease and kaliuretic peptide, ANP, and LANP caused a 31%, 31%, and 30% decrease in renal cancer cells, respectively (Fig. 4). At 96 hours, there was a 39% decrease with all four peptides while vessel dilator caused a 43% decrease and kaliuretic peptide, ANP, and LANP caused a 25%, 24%, and 24% decrease, respectively, at their 1 µM concentrations.

### Inhibition of DNA synthesis in renal cancer cells by urodilatin, LANP, vessel dilator, ANP and kahuretic peptide.

To help determine the mechanism of renal carcinoma cells' decrease in number and cellular proliferation by the above five hormones, the present study investigated if their effects were owing to an inhibition of DNA synthesis. Urodilatin, vessel dilator, LANP, katiuretic peptide and ANP each at their I µM concentrations inhibited DNA synthesis when incubated with the human renal carcinoma cells for 24 hours by 65%, 84%, 70%, 74%, and 77%, respectively (P<0.001 for each) (Fig. 5). Combination of the four cardiac peptides (each at 1 µM) resulted in an 82% decrease in DNA synthesis (Fig. 5).

### NPR-A and C-receptors are present in human renal carcinoma cells

Renal carcinoma cells have never been evaluated to determine whether they have NPR-A and/or -C receptors. When the human renal carcinoma cells were evaluated by Western blots, the NPR-A and -C receptors were demonstrated to be present (Fig. 6).

### DISCUSSION

This investigation is the first evidence that urodilatin has anticancer effects. Urodilatin structure is very similar to ANP with the 27 a.a. in ANP being identical in urodilatin and both have the same ring structure [27-32]. Urodilatin binds to the NPR-A and C receptors with binding curves superimposable with ANP [39-41]. Thus, based upon this knowledge, our hypothesis was that urodilatin would have similar anticancer effects to ANP. The present investigation indicates that these two peptides have very similar ability to decrease the number of renal cancer cells at their 100 µM concentrations (each at P<0.001). The potency of urodilatin's anticancer effects are most similar to ANP and kaliuretic peptide's from which it is derived by a different post-transplantational processing the ANP prohormone in the kidney [27-32].

Vessel dilator was the most potent of these peptide hormones in decreasing the number of human renal carcinoma cells at each of the respective concentrations of the peptide hormones (Fig. 2). In the dose-response curves of the present investigation, when vessel dilator concentration was increased 10-fold and 100-fold (i.e., 10 µM and 100 µM), vessel dilator decreased the number of human cancer cells 62% and 81%, respectively, compared to 59% decrease at its 1 µM concentration within 24 hours (Fig. 2). Vessel dilator also decreased human pancreatic [10], breast [12], colon [16] and prostate [15] adenocarcinomas as well as decreasing small-cell [14] and squamous lung cancer cells [13] *in vitro* the most. This information plus the knowledge that vessel dilator decreases the tumor volume of human pancreatic adenocarcinomas the most *in vivo* [11] suggests that vessel dilator has the most significant anticancer properties of the five peptide hormones in the present investigation. At each 10-fold increase in concentration of the respective peptide hormones in the present investigation (Fig. 2) vessel dilator's anticancer effects on renal carcinoma cells were more significant than the other four peptide hormones (P<0.05).

The remaining three peptide hormones synthesized by the cardiac ANP gene, however, had significant effects on decreasing the number of human renal carcinoma cells. When the concentration of kaliuretic peptide, ANP, and LANP were increased to 100 µM they caused a very significant 70-74% decrease in the number of renal carcinoma cells within 24 hours. There appears to be a difference in these peptide hormones ability to decrease cancer cell number depending on the type of cancer. Kaliuretic peptide's (1 µM), for example, ability to decrease the number of human renal carcinoma cells (39% decrease) is better than in most other cancers with a 30% decrease in prostate adenocarcinoma [15] and small-cell lung cancer cells [14] but its effects in the present investigation are similar to its effect on human pancreatic adenocarcinoma cells [10]. It is important to note that after 24 hours of incubation with the five peptide hormones that cellular debris was present, suggesting that cellular necrosis was occurring. Three days after no further exposure to these peptide hormones the renal cancer cells began to proliferate indicating that these peptide hormones need to be given more than a one-day exposure if one would hope to stop the growth of renal cell carcinomas. If, however, one gives these peptide hormones continuously to human cancer cells for four days, there is no proliferation of the cancer cells [10,12-16].

In the present investigation it was evaluated for the first time whether adding together simultaneously all four of the peptides synthesized by the cardiac ANP gene could cause a greater decrease than when each of these four peptide hormones we utilized individually. Combining kaliuretic peptide, ANP, LANP, and vessel dilator, each at 1 µM, caused a 58% decrease in renal carcinoma cell within 24 hours. Although this 58% decrease was larger than ANP (35%), kaliuretic peptide (39%) and LANP's (35%) decreases in cell number at their 1 µM concentrations for 24 hours, the combined decrease secondary to these four peptide hormones was not larger than that of vessel dilator (1 µM) used alone (i.e., 59% decrease). Similar findings were found at 48, 72 and 96 hours. The probable reason for this finding is that these four peptide hormones' major mechanism(s) of action in cancer cells is the same, i.e., they are strong inhibitors of DNA synthesis mediated specifically by cyclic GMP as evidenced by using a cyclic GMP antibody blocks all of these peptide hormones' effects on DNA synthesis in cancer cells [16].

Urodilatin and each of the four peptide hormones from the cardiac ANP prohormone inhibited 65-84% of the amount of DNA synthesis in the human renal carcinoma cells. We have previously demonstrated that the DNA synthesis-inhibiting properties of these peptide hormones synthesized by the cardiac ANP gene were directly due to the peptide hormones themselves as when their specific antibodies were incubated with the peptide hormones the antibodies completely blocked these peptide hormones ability to decrease cancer cell DNA synthesis [15]. The antibodies by themselves did not block DNA synthesis [15]. These findings suggest that one important mechanism of action of the five peptide hormones in the present investigation ability to inhibit cancer cell number is via their ability to inhibit DNA synthesis.

The present investigation is the first evaluation of whether renal carcinoma cells contain natriuretic peptide receptors. Both the NPR-A and NPR-C receptors were present in these human renal carcinoma cells. Since urodilatin binds to these receptors similar to ANP, with the two peptide having nearly superimposable binding curves [39-41], this helps to explain how urodilatin has effects in cancer cells, i.e., it binds to a specific cell surface receptor to help it to enter the cell where, as has been demonstrated for LANP, vessel dilator, ANP and kaliuretic peptide, it could localize to the nucleus of the cancer cell to directly inhibit DNA synthesis [42].

At present, standard therapy of renal carcinomas is radical nephrectomy which includes removal of the kidney en bloc with Gerota's fascia, as well as removal of the ipsilateral adrenal gland and regional lymph nodes [3]. Rates of response to chemotherapy for renal carcinomas are extremely low, i.e., roughly only four to six percent when 4,093 patients in eight clinical trials were evaluated with only 1% having a complete response [43].

With an estimated 12,600 renal cancer deaths in 2005 with surgery and current cancer chemotherapy plus radiation and immunotherapy [1] there is an urgent need to develop new approaches to therapy of renal carcinoma. The present investigation details not only one but five new potential therapies which kill up to 81% of human renal cancer cells within 24 hours. These five peptide hormones which circulate normally in the human body [44-50] have no known cytotoxic effects to normal cells [10] and only one known side effect [17-26]. This side effect, i.e., hypotension, has only been observed with ANP and urodilatin and never with vessel dilator, LANP, or kaliuretic peptide in human or animal subjects [18-26]. Present use of chemotherapy commonly causes toxicity in the form of nausea, vomiting, alopecia, and myelosuppression. None of these toxicities occur with the cardiac natriuretic peptide hormones or urodilatin [11,18-26].

In summary, FIG. 7 illustrates the inventive method. In step 1 renal carcinoma cells are identified, either *in vivo* or *in vitro.* In step 2 the proper peptide hormone (or combination of peptide hormones or as an adjunct to other chemotherapeutic agents), derived from the ANP prohormone including urodilatin, is selected and given to the patient. The target renal cells are then contacted with an effective amount (discussed *infra)* of the peptide hormone(s) in step 3. Finally, in step 4, the remaining carcinoma cells are quantified and the patient is evaluated for the need for further treatment.

It should be noted for the purposes of 35 U.S.C. §112, first paragraph, that urodilatin is comprised of identical amino acids to atrial natriuretic peptide and the last four n-termninal amino acids of kaliuretic peptide and has nearly identical anticancer effects on renal carcinoma cells as ANP. Urodilatin works through the same receptor as ANP and will have anticancer effects on all cancers that ANP has been shown to have significant (P<0.001) anticancer effects. These include the following: pancreatic, breast, prostate, colon and ovarian adenocarcinomas, medullary carcinoma of the thyroid, glioblastomas of the brain, angiosarcomas of the heart, melanomas, small-cell and squamous lung carcinomas. There is not a single cancer that ANP does not have significant anticancer effect upon and urodilatin likewise will have anticancer effects on all cancers.

### REFERENCES:

Jemal A, Murray T, Ward E, Samuels A, Tiwari RC, Ghafoor A, et al. Cancer statistics, 2005. CA Cancer J Clin 2005;55:10-30.
Chow WH, Devesa SS, Warren JL, Fraumeni JF Jr. Rising incidence of renal cell cancer in the United States. JAMA 1999;281:1628-1631.
Cohen HT, McGovern FJ. Renal-cell carcinoma. N Engl J Med 2005;353:2477-2490.
McLaughlin JK, Mandel JS, Blot WJ, Schuman LM, Mehl ES, Fraumenl JF Jr. A population-based case-control study of renal cell carcinoma. J Natl Cancer Inst 1984;72:275-284.
Yu MC, Mack TM, Hanisch R, Cicioni C, Henderson BE. Cigarette smoking, obesity, diuretic use, and coffee consumption as risk factors for renal cell carcinoma. J Natl Cancer Inst 1986;77:351-356.
LaVecchia C, Negri E, D'Avanzo B, Franceschi S. Smoking and renal cell carcinoma. Cancer Res 1990;50:5231-5233.
ShapiroJA, Williams MA, Weiss NS. Body mass index and risk of renal cell carcinoma. Epidemiology 1999;10:188-191.
Clark JI, Atkins MB, Urba WJ, Creech S, Figlin RA, Dutcher JP, et al. Adjuvant high-dose bolus interleukin-2 for patients with high-risk renal cell carcinoma: a cytokine working group randomized trial. J Clin Oncol 2003;21:3133-3140.
Yang JC, Sherry RM, Steinberg SM, Topalian SL, Schwartzentruber DJ, Hwu P, et al. Randomized study of high-dose and low-dose interleukin-2 in patients with metastatic renal cancer. J Clin Oncol 2003;21:3127-3132.
Vesely BA, McAfee Q, Gower WR Jr, Vesely DL. Four peptides decrease the number of human pancreatic adenocarcinoma cells. Eur J Clin Invest 2003;33:998-1005.
Vesely DL, Clark LC, Garces AH, McAfee QW, Soto J, Gower WR Jr. Novel therapeutic approach for cancer using four cardiovascular hormones. Eur J Clin Invest 2004;34:674-82.
Vesely BA, Song S, Sanchez-Ramos J, Fitz SR, Solivan SR, Gower WR Jr, et al. Four peptide hormones decrease the number of human breast adenocarcinoma cells. Eur J Clin Invest 2005;35:60-9.
Vesely BA, Fitz SR, Gower WR Jr, Vesely DL. Vessel dilator: Most potent of the atrial natriuretic peptides in decreasing the number and DNA synthesis of squamous lung cancer cells. Cancer Lett 2006;233:226-231.
Vesely BA, Song S, Sanchez-Ramos J, Fitz SR, Alli A, Solivan SR, et al. Five cardiac hormones decrease the number of human small-cell cancer cellos. Eur J Clin Invest 2005;35:388-398.
Vesely BA, Alli AA, Song S, Gower WR Jr, Sanchez-Ramos J, Vesely DL. Four peptide hormones specific decrease (up to 97%) of human prostate carcinoma cells. Eur J Clin Invest 2005;35:700-710.
Gower WR, Jr, Vesely BA, Alli AA, Vesely DL. Four peptides decrease human colon adenocarcinoma cell number and DNA synthesis via guanosine 3',5'-cyclic monophosphate. Int J Gastrointestinal Cancer 2005;36:77-88.
Vesely DL, Norris JS, Walters JM, Jespersen RR, Baeyens DA. Atrial natriuretic prohormone peptides 1-30,31-67 and 79-98 vasodilate the aorta. Biochem Biophys Res Commun 1987;148:1540-1548.
Martin DR, Pevahouse JB, Trigg DJ, Vesely DL, Buerkert JE. Three peptides from the ANF prohormone NH2-terminus are natriuretic and/or kaliuretic. Am J Physiol 1990;258:F1401-1408.
Gunning ME, Brady HR, Otuechere G, Brenner BM, Ziedel ML. Atrial natriuretic peptide (31-67) inhibits Na transport in rabbit inner medullary collecting duct cells: Role of prostaglandin E2. J Clin Invest 1992;89:1411-1417.
Benjamin BA, Peterson TV. Effects of proANF (31-67) on sodium excretion in conscious monkeys. Am J Physiol 1995;69:R1351-R1355.
Zeidel ML. Regulation of collecting duct Na+ reabsorption by ANP 31-67. Clin Exp Pharmacol Physiol 1995;22:121-124.
Villarreal D, Reams GP, Taraben A, Freeman RH. Hemodynamic and renal effects of proANF 31-67 in hypertensive rats. Proc Soc Exp Biol Med 1999;221:166-70.
Dietz JR, Scott DY, Landon CS, Nazian SJ. Evidence supporting a physiological role for proANP (1-30) in the regulation of renal excretion. Am J Physiol 2001;280:R1510-R1517.
Vesely DL, Douglass MA, Dietz JR, Gower WR Jr, McCormick MT, Rodriguez-Paz G, et al. Three peptides from the atrial natriuretic factor prohormone amino terminus lower blood pressure and produce diuresis, natriuresis and/or kaliuresis in humans. Circulation 1994;90:1129-1140.
Vesely DL, Douglass MA, Dietz JR, Giordano AT, McCormick MT, Rodriguez-Paz G, et al. Negative feedback of atrial natriuretic peptides. J Clin Endocrinol Metab 1994;78:1128-1134.
Vesely DL, Dietz JR, Parks JR, Baig M, McCormick MT, Cintron G, et al. Vessel dilator enhances sodium and water excretion and has beneficial hemodynamic effects in persons with congestive heart failure. Circulation 1998;98:323-329.
Vesely DL. Atrial Natriuretic Hormones. Englewood Cliffs NJ, Prentice Hall, 1992, 256 pp
Vesely DL. Natriuretic hormones, in The Kidney: Physiology and Pathophysiology, 4th edition, edited by Alpern RJ, Hebert SC, San Diego, CA, Elsevier/Academic Press, 2006, in press
Schulz-Knappe P, Forssmann K, Herbst F, Hock D, Pipkom R, Forsmann WG. Isolation and structural analysis of 'urodilatin', a new peptide of the cardiodilatin-(ANP)-family, extracted from human urine. Klin Wochenschr 1988;66:752-759.
Epstein M, Gerzer R. Natriuretic peptides and the kidney, inTextbook of Nephrology, edition 3, edited by Massry SG, Glassrock RD, Baltimore, Williams & Wilkins, 1995, pp 227-231
Vesely DL. Atrial natriuretic peptide prohormone gene expression: Hormones and diseases that upregulate its expression. IUBMB Life 2002;53:153-159.
Vesely DL. Natriuretic peptides and acute renal failure. Am J Physiol 2003;285:F167-177.
Vesely BA, Alli A, Song S, Sanchez-Ramos J, Fitz SR, Gower WR Jr, et al. Primary malignant tumors of the heart: Four cardiovascular hormones decrease the number and DNA synthesis of human angiosarcoma cells. Cardiology 2006;105:226-233.
Fisher ER, Palekar A, Paulson JD. Comparative histopathologic, histochemical, electron microscopy and tissue culture studies of bronchial carcinoids and oat cell carcinomas of lung. Am J Clin Pathol 1978;69:165-172.
Gratzner HG. Monoclonal antibody to 5-bromo-ad 5-lodo-deoxyuridine: A new reagent for detection of DNA replication. Science 1982;218:474-475.
Yu CCW, Woods AL, Levison DA. The assessment of cellular proliferation by immunohistochemistry: a review of currently available methods and their applications. Histochemical J 1992;24:121-131.
Morstyn G, Pyke K, Gardner J, Ashcroft R, deFazio A, Bhathal P. Immunohistochemical identification of proliferatory cells in organ culture using bromodeoxyuridine and a monoclonal antibody. J Histochem Cytochem 1986;34:697-701.
Qin Y, Williams G. Comparison of the classical autoradiographic and immunohistochemical methods with BrdU for measuring proliferation parameters in colon cancer. Anticancer Res 1993;13:731-736.
Heim JM, Kiefersauer S, Fülle HJ, Gener R. Urodilatin and beta-ANP: Binding properties and activation of particulate guanylate cyclase. Biochem Biophys Res Commun 1989;163:37-41.
Saxenhofer H, Fitzgibbon WR, Paul RV. Urodilatin: Binding properties and stimulation of cGMP generation in rat kidney cells. Am J Physiol 1993;264:F267-F273.
Valentin JP, Sechi LA, Qiu C, Schambelan, Humphrey MH. Urodilatin binds to and activates renal receptors for atrial natriuretic peptide. Hypertension 1993;21:432-438.
Saba SR, Garces AH, Clark LC, Soto J, Gower WR Jr, Vesely DL. Immunocytochemical localization of atrial natriuretic peptide, vessel dilator, long acting natriuretic peptide, and kaliuretic peptide in human pancreatic adenocarcinomas. J Histochem Cylochem 2005;53:989-995.
Yagoda A, Abi-Rached B, Petrylak D. Chemotherapy for advanced renal-cell carcinoma: 1983-1993. Semin Oncol 1995;22:42-60.
Winters CJ, Saliman AL, Baker BJ, Meadows J, Rico DM, Vesely DL. The N-terminus and a 4000 molecular weight peptide from the mid portion of the N-terminus of the atrial natriuretic factor prohormone each circulate in humans and increase in congestive heart failure. Circulation 1989;80:438-449.
Vesely DL, Norsk P, Winters CJ, Rico DM, Sallman AL, Epstein M. Increased release of the N-terminal and C-terminal, portions of the prohormone of atrial natriuretic factor during immersion-induced central hypervolemia in normal humans. Proc Soc Exp Biol Med 1989;192:230-235.
Hunter EFM, Kelly PA, Prowse C, Woods FJ, Lowry PJ. Analysis of peptides derived from pro atrial natriuretic peptide that circulate in man and increase in heart disease. Scan J Clin Lab Invest 1998;58:205-216.
Franz M, Woloszczuk W, Horl WH. N-terminal fragments of the proatrial natriuretic peptide in patients before and after hemodialysis treatment. Kidney Int 2000;58:374-378.
De Palo EF, Woloszczuk W, Meneghetti M, DePalo CB, Nielsen HB, Secher NH. Circulating immunoreactive proANP (1-30) and proANP (31-67) in sedentary subjects and athletes. Clin Chem 2000;46:843-847.
Franz M, Woloszczuk W, Horl WH. Plasma concentration and urinary excretion of N-terminal proatrial natriuretic peptides in patients with kidney diseases. Kidney Int 2001;59:1928-1934.
Vesely DL, Overton RM, Blankenship M, McCormick MT, Schocken DD. Atrial natriuretic peptide increases urodilatin in the circulation. Am J Nephrol 1998;18:204-213.

It will be seen that the advantages set forth above, and those made apparent from the foregoing description, are efficiently attained and since certain changes may be made in the above construction without departing from the scope of the invention, it is intended that all matters contained in the foregoing description or shown in the accompanying drawings shall be interpreted as illustrative and not in a limiting sense.

It is also to be understood that the following claims are intended to cover all of the generic and specific features of the invention herein described, and all statements of the scope of the invention which, as a matter of language, might be said to fall there between.

## Claims

1. Use of urodilatin for the manufacture of a medicament for inhibiting the growth of cancer cells.

2. Use of claim 1, wherein at least one other peptide hormone derived selected from the group consisting of atrial natriuretic peptide, long acting natriuretic peptide, vessel dilator, and kaliuretic peptide is used in conjunction with urodilatin.

3. Use of claim 1, wherein the cancer cells are renal carcinoma.

4. Use of a combination of peptide hormones derived from the atrial natriuretic peptide prohormone for the manufacture of a medicament for inhibiting the growth of cancer cells, wherein the combination includes urodilatin.

5. Use of claim 4, wherein the combination of peptide hormones derived from the atrial natriuretic peptide prohormone is selected from the group consisting of atrial natriuretic peptide, long acting natriuretic peptide, vessel dilator, and kaliuretic peptide.

6. Use of claim 4, wherein the cancer cells are renal carcinoma.

## Patentansprüche

1. Verwendung von Urodilatin zur Herstellung eines Medikaments zur Hemmung des Wachstums von Krebszellen.

2. Verwendung nach Anspruch 1, wobei in Verbindung mit Urodilatin mindestens ein weiteres abgeleitetes Peptidhormon verwendet wird, das aus der Gruppe bestehend aus dem atrialen natriuretischen Peptid, dem long-acting natriuretischen Peptid, Vasodilatator und dem kaliuretischen Peptid ausgewählt ist.

3. Verwendung nach Anspruch 1, wobei die Krebszellen Nierenkrebs sind.

4. Verwendung einer Kombination aus Peptidhormonen, die von dem Vorläufer des atrialen natriuretischen Peptidhormons abgeleitet sind, zur Herstellung eines Medikaments zur Hemmung des Wachstums von Krebszellen, wobei die Kombination Urodilatin enthält.

5. Verwendung nach Anspruch 4, wobei die Kombination aus Peptidhormonen, die von dem Vorläufer des atrialen natriuretischen Peptidhormons abgeleitet sind, aus der Gruppe bestehend aus dem atrialen natriuretischen Peptid, dem long-acting natriuretischen Peptid, Vasodilatator und dem kaliuretischen Peptid ausgewählt ist.

6. Verwendung nach Anspruch 4, wobei die Krebszellen Nierenkrebs sind.

## Revendications

1. Utilisation de l'urodilatine pour la fabrication d'un médicament pour inhiber la croissance des cellules cancéreuses.

2. Utilisation selon la revendication 1, dans laquelle au moins une autre hormone peptidique dérivée, choisie dans le groupe constitué du peptide natriurétique atrial, du peptide natriurétique à action prolongée, d'un vasodilatateur, et du peptide kaliurétique, est utilisée en conjonction à l'urodilatine.

3. Utilisation selon la revendication 1, dans laquelle les cellules cancéreuses sont des cellules de carcinome rénal.

4. Utilisation d'une combinaison d'hormones peptidiques dérivées de la prohormone peptidique natriurétique atriale pour la fabrication d'un médicament pour inhiber la croissance de cellules cancéreuses, la combinaison comprenant l'urodilatine.

5. Utilisation selon la revendication 4, dans laquelle la combinaison d'hormones peptidiques dérivées de la prohormone peptidique natriurétique atriale est choisie dans le groupe constitué du peptide natriurétique atrial, du peptide natriurétique à action prolongée, d'un vasodilatateur, et du peptide kaliurétique.

6. Utilisation selon la revendication 4, dans laquelle les cellules cancéreuses sont des cellules de carcinome rénal.
